Europäisches Patentamt

(19) European Patent Office  (11) Numéro de publication : **0 034 077**

Office européen des brevets  **B2**

(12)  # NOUVEAU FASCICULE DE BREVET EUROPÉEN

(45) Date de publication du nouveau fascicule du brevet :  (51) Int. Cl.⁴ : **H 01 L 41/08**, A 61 M 16/00,
22.10.86  A 61 B 5/10

(21) Numéro de dépôt : **81400100.4**

(22) Date de dépôt : **23.01.81**

(54) **Perfectionnements aux nappes composites constitutives de transducteurs électromécaniques et aux transducteurs équipés de telles nappes.**

(30) Priorité : **12.02.80 FR 8003093**

(43) Date de publication de la demande :
**19.08.81 Bulletin 81/33**

(45) Mention de la délivrance du brevet :
**05.10.83 Bulletin 83/40**

(45) Mention de la décision concernant l'opposition :
**22.10.86 Bulletin 86/43**

(84) Etats contractants désignés :
**BE CH DE GB IT LI NL SE**

(56) Documents cités :
**DE-A- 2 729 223**
**US-A- 3 944 763**
**US-A- 3 991 746**
**US-A- 3 996 922**
**US-A- 4 033 332**
**US-A- 4 041 446**
**US-A- 4 156 800**
**McGraw-Hill Encyclopedia of Science and Techno-logy. 1977. Vol. 14. p. 24 E -26**
**VDI-Z, Vol. 123, 1981, No. 20, octobre (II), pages 839-843 (République Fédérale d'Allemagne)**

(73) Titulaire : **Lewiner, Jacques**
**5, rue Bory d'Arnex**
**F-92210 Saint-Cloud (FR)**

**Hennion, Claude**
**18, rue Flatters**
**F-75005 Paris (FR)**

(72) Inventeur : **Lewiner, Jacques**
**5, rue Bory d'Arnex**
**F-92210 Saint-Cloud (FR)**
Inventeur : **Hennion, Claude**
**18, rue Flatters**
**F-75005 Paris (FR)**

(74) Mandataire : **Behaghel, Pierre et·al**
**CABINET PLASSERAUD 84 rue d'Amsterdam**
**F-75009 Paris (FR)**

Jouve, 18, rue St-Denis, 75001 Paris, France

## Description

L'invention est relative aux nappes composites constitutives de transducteurs électromécaniques et plus particulièrement à celles, de ces nappes, qui présentent une certaine souplesse et s'étendent sur une relativement grande surface, cette surface étant de préférence de l'ordre de plusieurs décimètres carrés.

Elle vise également les transducteurs équipés de telles nappes composites.

On sait que ces transducteurs permettent de transformer en variations d'une tension électrique les variations de pression qui sont exercées localement sur la nappe et/ou les déformations locales de cette nappe. Le brevet US-A 3 996 922 constitue un exemple d'une telle nappe.

Les nappes composites en question comprennent essentiellement à cet effet une feuille diélectrique « sensible » à la pression au sens de la transduction électromécanique, feuille chargée électriquement en permanence ou porteuse d'une polarisation électrique permanente, interposée entre aux électrodes ou pellicules conductrices de l'électricité elles-mêmes recouvertes extérieurement de deux feuilles de protection, et les transducteurs équipés de ces nappes composites comportent, en plus de celles-ci, des moyens électriques d'exploitation et éventuellement d'alarme connectés aux deux électrodes des dites nappes composites.

Une application intéressante de ces transducteurs, à laquelle l'invention se rapporte de préférence, mais non exclusivement, est la détection des apnées, la surveillance des fonctions biologiques (respiration, rythme cardiaque, ...), en particulier chez les nourrissons.

On constitue à cet effet, à l'aide des nappes composites considérées, des alèses que l'on interpose entre un matelas et le sujet à surveiller, couché, assis ou appuyé sur le matelas : les mouvements respiratoires de ce sujet provoquent des variations locales cycliques de la pression exercée sur cette alèse, variations qui se traduisent par des variations cycliques semblables d'une tension électrique, et les moyens d'exploitation sont agencés de façon à exciter l'alarme lorsque lesdites variations cycliques de tension cessent pendant une durée qui dépasse un seuil estimé dangereux ou présentent une forme s'écartant dans une mesure excessive d'une forme considérée comme normale.

L'invention a pour but, surtout, d'améliorer la fidélité et la fiabilité des transducteurs du genre en question.

A cet effet,

— dans le cas où la feuille diélectrique sensible à la pression est constituée en un matériau diélectrique porteur de charges permanentes et créant un champ électrique extérieur, les nappes composites du genre ci-dessus sont essentiellement caractérisées en ce qu'elles comprennent, directement, entre la feuille sensible et l'une des deux électrodes, des cales d'écartement dont les deux faces opposées sont collées respectivement sur les deux plages adjacentes en regard,

— et dans le cas où la feuille diélectrique sensible à la pression est constituée en un matériau diélectrique piézo-électrique porteur d'une polarisation électrique permanente, les nappes composites du genre en question sont essentiellement caractérisées en ce que, ladite feuille étant interposée jointivement entre les deux électrodes, lesdites nappes comprennent entre l'une de ces électrodes et la feuille de protection extérieure correspondante, des cales d'écartement dont les deux faces opposées sont collées respectivement sur les deux plages adjacentes en regard.

Dans des modes de réalisation préférés, on a recours en outre à l'une et/ou à l'autre des dispositions suivantes :

— les cales sont prévues autocollantes sur leurs deux faces,

— les plages sur lesquelles sont collées les cales sont traitées préalablement de manière à faciliter ce collage, notamment par bombardement électronique ou ionique, ou encore par dépôt d'une couche mince d'un matériau, tel que le chrome, offrant une bonne prise aux colles,

— les cales sont constituées par une grille,

— les cales sont constituées par une feuille continue perforée,

— les cales sont constituées par des bandelettes parallèles,

— les cales sont constituées par des plots espacés,

— les cales ont une épaisseur comprise entre 0,1 et 1 mm, une largeur comprise entre 1 et 10 mm et un espacement mutuel compris entre 5 et 100 mm,

— la nappe composite constitue une alèse rectangulaire pour nourrissons dont l'un des côtés est compris entre 10 et 30 cm et l'autre côté entre 20 et 50 cm.

L'invention comprend, mises à part ces dispositions principales, certaines autres dispositions qui s'utilisent de préférence en même temps et dont il sera plus explicitement question ci-après.

Dans ce qui suit, l'on va décrire deux modes de réalisation préférés de l'invention en se référant aux dessins ci-annexés d'une manière bien entendu non limitative.

La figure 1 de ce dessin, montre en vue perspective, portions arrachées, une alèse établie conformément à l'invention.

La figure 2 montre à plus grande échelle, en coupe transversale, un morceau de cette alèse.

La figure 3 montre à encore plus grande échelle, en coupe transversale, un détail dudit morceau.

La figure 4 montre une variante d'un morceau d'alèse également conforme à l'invention et semblable à celle de la figure 2.

On fait comprendre à l'alèse A une feuille diélectrique 1 interposée entre deux feuilles, pellicules ou couches conductrices de l'électricité 2 et 3, désignées dans la suite par le mot « électrodes ».

La feuille 1 est choisie de façon telle qu'elle soit « sensible » au sens de la transduction électromécanique, c'est-à-dire qu'elle soit capable de transformer les variations de pression appliquées localement sur elle en variations de la tension électrique recueillie aux bornes des deux électrodes 2 et 3.

A cet effet, elle est avantageusement constituée en une matière plastique telle que le polypropylène, qu'un P.T.F.E., qu'un poly(éthylène-propylène fluoré) ou encore qu'un polymère ou copolymère à base de fluorure de polyvinylidène, son épaisseur étant de préférence comprise entre 5 et 50 microns.

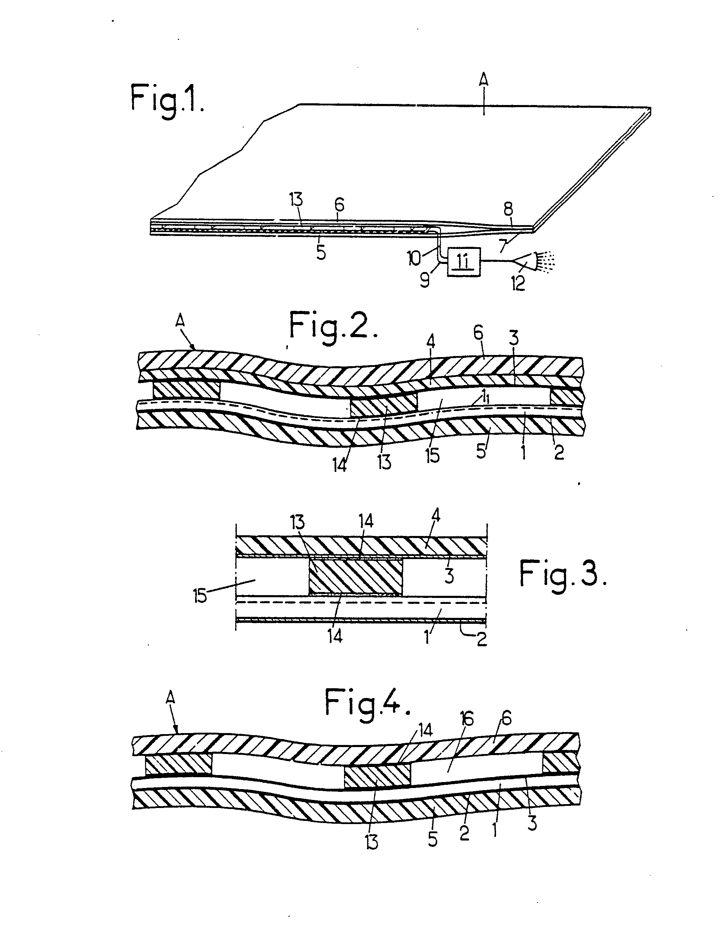

Cette feuille 1 est porteuse d'une polarisation électrique permanente ou chargée électriquement en permanence au moins au voisinage d'une $1_1$ de ses faces, par des charges positives et/ou négatives qui ont été schématisées par le signe — sur la figure 2.

Dans ce dernier cas (charge électrique permanente), la feuille 1 crée un champ électrique extérieur et elle est maintenue écartée, au moins en partie, de l'une des électrodes 3 par des intervalles isolants, comme visible sur la figure 2.

Dans l'autre cas (polarisation électrique permanente), schématisé sur la figure 4, la feuille 1 possède des propriétés piézo-électriques et elle est enveloppée jointivement par les deux électrodes 2 et 3.

Chacune des électrodes 2 et 3 est avantageusement constituée par métallisation superficielle d'une feuille en matière plastique.

Dans le cas de la feuille sensible chargée (figure 2), l'une, 2, de ces électrodes est rapportée sur cette feuille 1 alors que l'autre électrode 3, maintenue légèrement écartée de ladite feuille 1 par tous moyens désirables, est rapportée sur une feuille plastique supplémentaire 4.

Dans le cas de la feuille sensible 1 polarisée (figure 4), les deux électrodes 2 et 3 sont rapportées sur respectivement les deux faces de cette feuille 1.

L'ensemble des trois feuilles 1, 2 et 3 est lui-même disposé entre deux feuilles de protection extérieures 5 et 6, dont l'épaisseur est avantageusement comprise entre 0,1 et 0,5 mm, constituées en une matière souple isolante de l'électricité.

Ces feuilles de protection 5 et 6 débordent au-delà de l'ensemble des trois feuilles 1, 2 et 3 et leurs marges débordantes 7 et 8 (figure 1) sont assemblées l'une contre l'autre d'une manière étanche vis-à-vis des impuretés, notamment par collage ou soudage à chaud.

L'alèse A formée par la nappe souple composée des feuilles superposées 1, 2, 3, 5 et 6, présente la forme générale d'un rectangle dont les côtés mesurent respectivement entre 10 et 30 cm et entre 20 et 50 cm s'il s'agit d'une alèse pour nourrissons.

Chacune des électrodes 2 et 3 est reliée à l'aide de conducteurs 9 et 10 à un ensemble d'exploitation approprié 11 — avantageusement d'un type électronique à circuits C-MOS — propre à exciter un dispositif d'alarme 12 sonore, visuel ou autre.

Pour l'application de l'alèse considérée A à la détection des apnées d'un sujet reposant sur cette alèse, l'excitation de l'alarme 12 est automatiquement assurée lorsque les oscillations, de la tension recueillie entre les électrodes 2 et 3, dues à la respiration normale dudit sujet, s'interrompent pendant une durée supérieure à un seuil prédéterminé, lequel peut être réglé à volonté et être égal par exemple à 10 ou 30 secondes ou présentent une forme s'écartant dans une mesure excessive d'une forme considérée comme normale.

On prévoit en outre, conformément à l'invention, une pluralité de cales d'écartement 13 interposées entre la feuille sensible 1 et l'une, 6, des feuilles de protection extérieure, les deux faces opposées des cales étant collées respectivement sur les plages de feuille contre lesquelles elles sont juxtaposées.

Les cales 13 en question peuvent être réalisées en toutes formes et matières désirables.

Elles peuvent se présenter sous la forme de grilles ou treillis formés d'éléments croisés plats réunis de préférence à leurs points de croisement de façon à ne pas créer de surépaisseurs locales, ou encore sous la forme d'une feuille continue perforée, ou sous celle de bandelettes plates parallèles, ou de plots plats espacés, de section droite carrée, rectangulaire, circulaire ou autre.

Leur matériau constitutif peut être isolant ou conducteur de l'électricité : il est alors choisi de façon telle que les cales ne risquent pas de déchirer les feuilles souples contre lesquelles elles sont collées.

Dans ce même souci d'éviter de telles déchirures, il pourra être avantageux dans certains cas d'éviter les arêtes ou pointes trop vives sur les cales.

Les dimensions de ces cales 13, considérées selon un plan perpendiculaire à celui de la nappe composite tel que celui dans lequel est tracée la figure 3, sont de préférence comprises

— entre 0,1 et 1 mm pour l'épaisseur,
— entre 1 et 10 mm pour la largeur,
— et entre 5 et 100 mm pour l'espacement mutuel.

Le collage des cales 13 contre les plages de feuilles en regard est assuré à l'aide d'une mince couche de matière adhésive 14.

Une telle couche est avantageusement rapportée à l'avance sur chaque face de cale 13, par exemple par pulvérisation, de façon telle que la mise en place de ces cales puisse être réalisée très facilement par autocollage.

Vu leurs épaisseurs très faibles, par exemple de l'ordre de quelques dizaines de microns, les électrodes 2, 3 et couches adhésives 14 ont été représentées par des traits gras sur les figures 2 et 4 : la figure 3 à plus grande échelle permet de mieux les voir.

Pour faciliter le collage des cales 13 sur les plages de feuilles en regard, il peut être avantageux de traiter préalablement ces plages, notamment par bombardement électronique ou ionique (décharge électrique), ou encore par dépôt local (sous vide ou autrement) d'une couche mince d'un matériau, tel que le chrome, offrant une bonne compatibilité avec les colles.

Dans le premier mode de réalisation relatif à l'utilisation d'une feuille sensible chargée 1, qui a été illustrée sur les figures 2 et 3, les cales 13 sont directement interposées entre la face chargée $1_1$ de cette feuille et l'électrode 3 en regard : ce sont ces cales qui maintiennent écartées la face $1_1$ de l'électrode 3 et définissent entre elles des chambres ou compartiments 15 bien délimités.

Dans le second mode de réalisation relatif à l'utilisation d'une feuille 1 piézo-électrique polarisée, qui a été illustré sur la figure 4, les cales 13 sont interposées directement entre l'une, 3, des électrodes rapportées contre cette feuille 1 et la feuille de protection 6 correspondante, définissant encore entre ces éléments des chambres ou compartiments 16 bien délimités.

Dans chaque cas, le double collage des cales selon l'invention assure un fonctionnement particulièrement fidèle du transducteur :

— dans le premier cas, on est sûr que les variations de la distance entre la face chargée $1_1$ et l'électrode 3 en regard sont dues exclusivement aux déformations, de cet électret et/ou de cette électrode, qui se développent au niveau des différents compartiments 15 délimités entre les cales successives : en d'autres termes, il n'est pas question ici que les déformations individuelles exploitées aux fins de surveillance intéressent des portions de surface, de la nappe composite, couvrant des pluralités de cales voisines, voire des nombres variables de telles cales, comme ce serait le cas s'il y avait possibilité de décollage entre ces cales et les plages des feuilles contre lesquelles elles sont juxtaposées ;

— dans le second cas, les variations de pression exercées localement sur la feuille 1 se traduisent par des enfoncements élastiquement réversibles des portions, de cette feuille, disposées en regard des compartiments 16, à l'intérieur de ces compartiments, ce qui allonge, et donc amincit ces portions : ce sont surtout ces amincissements réversibles qui sont transformés en variations électriques exploitées aux fins de surveillance ; grâce au collage considéré, lesdits amincissements correspondent uniquement aux enfoncements signalés alors qu'une absence de collage des cales rendrait possibles des glissements de la feuille le long de ces cales, propres à dénaturer les caractéristiques de ces enfoncements.

Comme il va de soi, et comme il résulte d'ailleurs déjà de ce qui précède, l'invention ne se limite nullement à ceux de ses modes d'application et de réalisation qui ont été plus spécialement envisagés ; elle en embrasse, au contraire, toutes les variantes.

En particulier, les cales d'écartement pourraient être venues de moulage avec l'une des feuilles contre lesquelles elles sont destinées à être collées, formant par exemple des nervures en saillie sur la face intérieure de la figure 4.

De même, les cales d'écartement pourraient être agencées de façon à permettre un écoulement de l'air de l'extérieur de la nappe composite vers les alvéoles 15 et dans le sens inverse.

De même, l'une quelconque des deux structures composites décrites ci-dessus pourrait être doublée :

— on pourrait à cet effet faire comprendre en commun l'électrode 3 de la figure 2 par deux structures du type de celle illustrée sur ladite figure 2 et rapporter symétriquement deux ensembles cales-électret-autre électrode-feuille de protection sur respectivement les deux faces de cette électrode 3, cette dernière pouvant être elle-même dédoublée et constituée par métallisation des deux faces de la feuille supplémentaire 4,

— on pourrait également rapporter sur chacune des faces des cales 13 de la figure 4 l'ensemble d'une feuille 1 métallisée sur ses deux faces et revêtue extérieurement d'une nappe de protection.

Dans chacune de ces deux dernières variantes, les deux électrodes extérieures de la structure « doublée » obtenue peuvent être connectées ensemble et constituer un blindage électrostatique pour la nappe composite.

Dans une autre variante, les diverses couches de la nappe composite peuvent être constituées de matériaux transparents, de façon à ce que la nappe composite soit elle-même transparente.

Dans une autre variante, les électrodes 2 et 3 sont divisées en plusieurs plages électriquement disjointes, de façon à permettre une localisation sur la nappe composite de la région qui est déformée et où s'exerce la pression.

**Revendications**

1. Nappe composite relativement souple et étendue, constitutive d'un transducteur électromécanique et comprenant une feuille sensible à la pression au sens de la transduction électromécanique, ladite feuille sensible (1), constituée en un matériau diélectrique porteur de charges électriques permanentes et créant un champ électrique extérieur, étant interposée entre deux feuilles conductrices ou électrodes (2, 3) elles-mêmes recouvertes extérieurement de feuilles de protection (6), caractérisée en ce qu'elle comprend en outre, directement, entre la feuille sensible (1) et l'une des deux électrodes (3), des cales d'écartement (13) dont les deux faces opposées sont collées respectivement sur les deux plages adjacentes en regard.

2. Nappe composite relativement souple et étendue, constitutive d'un transducteur électromécanique et comprenant une feuille sensible à la pression au sens de la transduction électromécanique, ladite feuille sensible (1) étant constituée en un matériau diélectrique piézo-électrique porteur d'une polarisation électrique permanente et étant interposée entre deux feuilles conductrices ou électrodes elles-mêmes recouvertes extérieurement de feuilles de protection, caractérisée en ce que, ladite feuille étant interposée jointement entre les deux électrodes (2, 3), ladite nappe comprend en outre, entre l'une de ces électrodes (3) et la feuille de protection extérieure correspondante (6), des cales d'écartement (13) dont les deux faces opposées sont collées respectivement sur les deux plages adjacentes en regard.

3. Nappe selon l'une quelconque des précédentes revendications, caractérisée en ce que les cales (13) sont prévues autocollantes sur leurs deux faces.

4. Nappe selon l'une quelconque des précédentes revendications, caractérisée en ce que les plages sur lesquelles sont collées les cales (13) sont traitées préalablement de manière à faciliter ce collage, notamment par décharge électrique ou dépôt d'une couche mince appropriée.

5. Nappe selon l'une quelconque des précédentes revendications, caractérisée en ce que les cales sont constituées par une grille.

6. Nappe selon l'une quelconque des précédentes revendications, caractérisée en ce que les cales sont constituées par une feuille continue perforée.

7. Nappe selon l'une quelconque des précédentes revendications, caractérisée en ce que les cales sont constituées par des bandelettes parallèles.

8. Nappe selon l'une quelconque des précédentes revendications, caractérisée en ce que les cales sont constituées par des plots espacés.

9. Nappe selon l'une quelconque des précédentes revendications, caractérisée en ce que les cales ont une épaisseur comprise entre 0,1 et 1 mm, une largeur comprise entre 1 et 10 mm et un espacement mutuel compris entre 5 et 100 mm.

10. Alèse rectangulaire caractérisée en ce qu'elle est constituée par une nappe composite selon l'une quelconque des précédentes revendications, dont les côtés sont compris respectivement entre 10 et 30 cm et entre 20 et 50 cm.

11. Nappe selon l'une quelconque des précédentes revendications, caractérisée en ce que ses diverses couches sont constituées de matériaux transparents.

12. Nappe selon l'une quelconque des précédentes revendications, caractérisée en ce que les électrodes sont divisées en plusieurs plages électriquement disjointes.

13. Transducteur électromécanique caractérisé en ce qu'il comprend une nappe composite selon l'une quelconque des précédentes revendications et des moyens d'exploitation (11) et éventuellement d'alarme (12) connectés électriquement aux deux électrodes (2, 3) de cette nappe.

**Claims**

1. Relatively flexible and extended composite sheet, constituting an electromechanical transducer and comprising a foil sensitive to pressure in the sense of electromechanical transduction, said sensitive foil (1), made of a dielectric material carrying permanent electric charges and generating an external electric field, being interposed between two conductive foils or electrodes (2, 3) themselves covered externally with protective foils (6), characterized in that it further comprises, directly, between the sensitive foil (1) and one of the two electrodes (3), spacing shims (13) of which the two opposite surfaces are glued respectively to the two opposite adjacent areas.

2. Relatively flexible and extended composite sheet, constituting an electromechanical transducer and comprising a foil sensitive to pressure in the sense of electromechanical transduction, said sensitive foil (1) being made of a dielectric piezo-electric material carrying a permanent electric polarization and being interposed between two conductive foils or electrodes themselves covered externally with protective foils, characterized in that, said foil being contiguously interposed between the two electrodes (2, 3), said sheet further comprises, between one of said electrodes (3) and the corresponding external protection foil (6), spacing shims (13) of which the two opposite surfaces are glued respectively to the opposite adjacent areas.

3. Sheet according to anyone of the preceding claims, characterized in that the shims (13) are self-adhesive on their two surfaces.

4. Sheet according to anyone of the preceding claims, characterized in that the areas on which the shims (13) are glued are treated previously so as to facilitate this gluing, advantageously by electrical discharge or by deposition of a suitable thin layer.

5. Sheet according to anyone of the preceding claims, characterized in that the shims are constituted by a grid.

6. Sheet according to anyone of the preceding claims, characterized in that the shims are constituted by a perforated continuous foil.

7. Sheet according to anyone of the preceding claims, characterized in that the shims are constituted by parallel strips.

8. Sheet according to anyone of the preceding claims, characterized in that the shims are constituted by spaced studs.

9. Sheet according to anyone of the preceding claims, characterized in that the shims have a thickness comprised between 0.1 and 1 mm, a width comprised between 1 and 10 mm and a mutual spacing comprised between 5 and 100 mm.

10. Rectangular mattress construction, characterized in that it is constituted by a composite sheet according to anyone of the preceding claims, whose sides are comprised respectively between 10 and 30 cm and between 20 and 50 cm.

11. Sheet according to anyone of the preceding claims, characterized in that its various layers are constituted of transparent materials.

12. Sheet according to anyone of the preceding claims, characterized in that the electrodes are divided into several electrically disjointed areas.

13. Electromechanical transducer, characterized in that it comprises a composite sheet according to anyone of the preceding claims, and output means (11) and eventually alarm means (12) connected electrically to the two electrodes (2, 3) of said sheet.

**Patentansprüche**

1. Zusammengesetzte Folie, die verhältnismäßig biegsam und langgestreckt ist, die ein elektromechanischer Wandler bildet und eine in bezug auf die elektromechanische Umwandlung druckempfindliche Folie enthält, wobei die auf Druck ansprechende Folie (1), die aus einem dielektrischen Material besteht, das Träger permanenter elektrischer Ladungen ist und ein äußeres elektrisches Feld erzeugt, zwischen zwei leitenden Folien oder Elektroden (2, 3) angeordnet ist, die außen mit Schutzfolien (6) versehen sind, dadurch gekennzeichnet, daß die zusammengesetzte Folie darüber hinaus Abstandshalter (13) enthält, die direkt zwischen der auf Druck ansprechenden Folie (1) und einer der beiden Elektroden (3) angeordnet sind und daß die beiden sich gegenüberliegenden Oberflächen der Abstandshalter mit den beiden ihnen benachbarten Flächen verklebt sind.

2. Zusammengesetzte Folie, die verhältnismäßig biegsam und langgestreckt ist, die ein elektromechanischer Wandler bildet und eine in bezug auf die elektromechanische Umwandlung druckempfindliche Folie enthält, wobei die auf Druck ansprechende Folie (1) aus einem dielektrischen piezoelektrischen Material besteht, das Träger einer permanenten elektrischen Polarisation ist und zwischen zwei leitenden Folien oder Elektroden angeordnet ist, die außen mit Schutzfolien versehen sind, dadurch gekennzeichnet, daß die Folie dicht zwischen den beiden Elektroden (2, 3) angeordnet ist und die zusammengesetzte Folie darüber hinaus Abstandshalter (13) enthält, die zwischen einer der Elektroden (3) und der entsprechenden äußeren Schutzfolie (6) angeordnet sind, wobei die sich gegenüberliegenden Oberflächen der Abstandshalter mit den beiden ihnen benachbarten Flächen verklebt sind.

3. Zusammengesetzte Folie nach einem der vorhergehenden Ansprüche, dadurch gekennzeichnet, daß die Abstandshalter (13) an ihren beiden Oberflächen selbstklebend sind.

4. Zusammengesetzte Folie nach einem der vorhergehenden Ansprüche, dadurch gekennzeichnet, daß die Flächen, mit denen die Abstandshalter (13) verklebt werden, um das Ankleben zu erleichtern, insbesondere durch elektrische Entladung oder Aufbringen einer geeigneten dünnen Schicht, vorbehandelt sind.

5. Zusammengesetzte Folie nach einem der vorhergehenden Ansprüche, dadurch gekennzeichnet, daß die Abstandshalter aus einem Gitterwerk bestehen.

6. Zusammengesetzte Folie nach einem der vorhergehenden Ansprüche, dadurch gekennzeichnet, daß die Abstandshalter aus einer zusammenhängenden perforierten Folie bestehen.

7. Zusammengesetzte Folie nach einem der vorhergehenden Ansprüche, dadurch gekennzeichnet, daß die Abstandshalter aus parallel verlaufenden Riemchen bestehen.

8. Zusammengesetzte Folie nach einem der vorhergehenden Ansprüche, dadurch gekennzeichnet, daß die Abstandshalter aus im Abstand voneinander angeordneten Klötzen bestehen.

9. Zusammengesetzte Folie nach einem der vorhergehenden Ansprüche, dadurch gekennzeichnet, daß die Abstandshalter eine zwischen 0,1 und 1 mm liegende Stärke, eine zwischen 1 und 10 mm liegende Breite und einen Abstand voneinander haben, der zwischen 5 und 100 mm liegt.

10. Rechteckige Unterlage, dadurch gekennzeichnet, daß sie aus einer zusammengesetzten Folie nach einem der vorhergehenden Ansprüche besteht, deren Seitenlängen zwischen 10 und 30 cm bzw. 20 und 50 cm liegen.

11. Zusammengesetzte Folie nach einem der vorhergehenden Ansprüche, dadurch gekennzeichnet, daß die verschiedenen Schichten aus transparentem Material bestehen.

12. Zusammengesetzte Folie nach einem der vorhergehenden Ansprüche, dadurch gekennzeichnet, daß die Elektroden in mehrere elektrisch voneinander getrennte Flächen aufgeteilt sind.

13. Elektromechanischer Wandler, dadurch gekennzeichnet, daß er eine zusammengesetzte Folie gemäß einem der vorhergehenden Ansprüche, sowie Einrichtungen (11) zur Auswertung und gegebenenfalls Alarmeinrichtungen (12) enthält, die elektrisch mit den zwei Elektroden (2, 3) der zusammengesetzten Folie verbunden sind.

Fig.1.

Fig.2.

Fig.3.

Fig.4.